(19) Europäisches Patentamt
European Patent Office
Office européen des brevets

(11) EP 0 687 250 B1

(12) EUROPEAN PATENT SPECIFICATION

(45) Date of publication and mention of the grant of the patent:
**23.10.2002 Bulletin 2002/43**

(21) Application number: **94909515.2**

(22) Date of filing: **27.01.1994**

(51) Int Cl.[7]: **C07D 207/38**, C08F 26/08

(86) International application number:
**PCT/US94/01075**

(87) International publication number:
**WO 94/020461 (15.09.1994 Gazette 1994/21)**

(54) **Method for the preparation of 1-VINYL-3(E)-ETHYLIDENE PYRROLIDONE**

Verfahren zur Herstellung von 1-VINYL-3(E)-ETHYLIDEN PYRROLIDON

Procédé pour la préparation de1-VINYL-3(E)-ETHYLIDENE PYRROLIDONE

(84) Designated Contracting States:
**DE**

(30) Priority:
**03.03.1993 US 25523**
**30.03.1993 US 40400**
**31.03.1993 US 40805**
**31.03.1993 US 40806**
**31.03.1993 US 40807**
**06.05.1993 US 57378**

(43) Date of publication of application:
**20.12.1995 Bulletin 1995/51**

(73) Proprietor: **ISP INVESTMENTS INC.**
**Wilmington, Delaware 19801 (US)**

(72) Inventors:
- **TSENG, Susan, Y.**
**Staten Island, NY 10306 (US)**
- **WOLF, Philip, F.**
**Bridgewater, NJ 08807 (US)**

(74) Representative: **Ford, Michael Frederick et al**
**MEWBURN ELLIS**
**York House**
**23 Kingsway**
**London WC2B 6HP (GB)**

(56) References cited:
**US-A- 5 274 120** **US-A- 5 286 876**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 687 250 B1

Printed by Jouve, 75001 PARIS (FR)

## Description

This invention relates to a method for obtaining the isomeric compound 1-vinyl-3(E)-ethylidene pyrrolidone (EVP). The compound is used for making crosslinked polyvinylpyrrolidone.

Crosslinked polyvinylpyrrolidone is made by popcorn or proliferous polymerization of vinylpyrrolidone, in the absence or presence of crosslinking agents, as described in U.S. Patents 3,277,066; 3,306,886; 3,759,880; 3,933,766; and 3,992,562; and in an article by F. Haaf et al. in Polymer J. 17 (1), p. 143-152 (1985), entitled, "Polymers of N-Vinylpyrrolidone: Synthesis, Characterization and Uses." Polymerization of vinylpyrrolidone can occur in the absence of added crosslinker because the requisite crosslinker in the process is formed in situ during the first stage heating of vinylpyrrolidone in aqueous caustic solutions at temperatures > 100° C., e.g. at 140°C. Such bifunctional monomers, identified as 1-vinyl-3-ethylidene pyrrolidone and ethylidene-bis-3-(N-vinylpyrrolidone), are observed by gas chromatography and other analytical techniques to be present in small amounts in reaction mixtures which had been cooled to room temperature. However, after the polymerization was completed, these bifunctional compounds, could not be found in the final product. Accordingly, the named bifunctional monomers are present only in small amounts as intermediates during the polymerization and are consumed in the process of forming the crosslinked PVP polymer.

The method provided herein can yield the isomeric compound 1-vinyl-3(E)-ethylidene pyrrolidone (EVP) having the formula:

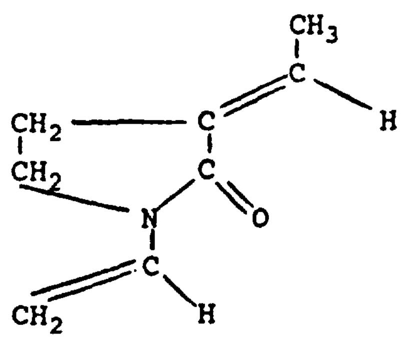

in a purity of at least 95%, in the form of white, needle-shaped crystals having a melting point of 59-61°C.

This isomeric compound exists in the (E) form, which is defined as the isomer in which the methyl group of the ethylidene radical is positioned away from the oxygen atom of the pyrrolidone ring.

The method involves reaction of vinylpyrrolidone in aqueous strongly basic solution, in a 2-phase aqueous-organic system, at an elevated temperature, under vigorous agitation. The isomeric compound is recovered from the reaction product by extraction with an organic solvent and fractional distillation, or by direct fractional distillation of the organic fraction of the reaction product. It may then be recrystallized to yield the pure compound.

In accordance with the present invention, the desired isomeric EVP compound is produced in a 2-phase reaction mixture comprising an organic phase of vinylpyrrolidone monomer present in an amount of about 25-90%, preferably 40-75%, and, most preferably, about 50%, by weight of the reaction mixture, and an aqueous phase which is a strongly aqueous basic solution, such as caustic (NaOH or KOH), or a tetraalkyl ammonium hydroxide solution, suitably with a base concentration of about 2-50%, preferably about 5-10%, by weight. The reaction mixture is heated to a reaction temperature of about 120-170°C., preferably 130-140°C., in a closed system, under an inert atmosphere, at ordinary or higher initial pressures, suitably at an initial pressure of 0-300 kPa (0-3 bars) of an inert gas, such as nitrogen. The reaction to convert VP monomer to isomeric EVP compound is carried out for about 0.5-10 hours, preferably 1-3 hours, at, e.g. 140°c., while the reaction mixture is subjected to vigorous agitation, e.g. about 800 rpm.

At the conclusion of the reaction, 2 layers are obtained as the reaction product. The top layer is an organic layer which contains about 50-80% by weight of unreacted VP and about 5-30% of the desired isomeric EVP compound, and, more particularly, about 70-75% VP and 15-20% EVP. The bottom layer is an aqueous caustic layer which also contains some some unreacted VP and a small amount of EVP.

The isomeric compound then is isolated from the reaction product by extraction with an organic solvent and fractional distillation, or by direct fractional distillation of the organic (top) layer.

In the extraction technique, the reaction product is treated with a volatile organic solvent, such as chloroform, heptane, or petroleum ether, and the extract is washed successively with a saturated salt solution, then distilled water, and the volatile organic solvent is removed by distillation under reduced pressure. The residue is distilled at 60-90°C. (1-2 mm Hg) to remove unreacted vinylpyrrolidone monomer and by-products of the reaction, including 2-pyrrolidone.

Then, at 80°-90°C., a second fraction is collected as a solid. Upon recrystallization of the solid product from water and drying, the desired isomeric (E) compound is obtained as white, needle-shaped crystals having a melting point of 59-61°C. The (E) isomeric chemical structure is confirmed by gas chromatographic, mass spectroscopy and $^1H_2$ and $^{13}C$ NMR analysis.

In the direct distillation method of recovery of the isomeric compound from the reaction mixture, the organic phase, which is the top layer, is separated from the aqueous caustic solution, which is the bottom layer, and then the organic layer is flash distilled under vacuum. A suitable vacuum distillation apparatus includes a receiving condenser connected to the distilling head at one end and to a receiving flask at the other end. The unreacted VP and 2-pyrrolidone by-products are distilled out from the organic layer as a clear liquid at 75-90°C. at 2.66 x $10^2$ Pa (2 mm Hg). The isomeric compound then is flash distilled as a colorless solid which crystallizes on the walls of the receiving condenser. This solid can be collected from the walls, or, preferably, the walls are heated to melt the solid to a liquid, and the liquid is recovered and solidified. Upon recrystallization from water, or from a water-acetone mixture, the desired isomeric compound is obtained as pure solid.

The process of rapid and efficient production of EVP in large quantities herein is based on the following two interdependent parameters.

(1) An initial high concentration of caustic catalyst in the reaction mixture, and

(2) Maintenance of a two-phase organic/aqueous system in the reaction mixture throughout the course of the reaction.

The use of a high (2-50%) caustic concentration has a dual effect. First the inorganic hydroxide causes the aqueous layer to maintain its integrity and "salt out" the organic compounds, most notably, vinylpyrrolidone (VP). Such is not the case for conventional PVP syntheses using a low concentration caustic solution in which the aqueous and organic phases merge. Secondly, the high caustic concentration in this process accelerates the reaction. Indeed, the caustic, which is a catalyst for the formation of EVP from VP, is consumed through reaction with 2-pyrrolidone, a by-product of the reaction. The 2-pyrrolidone, in turn, is readily hydrolyzed by base to sodium 4-aminobutyrate (4-AB), which is not a catalyst for EVP formation. However, (4-AB), being water soluble, can serve as the salt necessary to maintain the 2-phase system in the process.

During the process, transfer of the vinyl moiety which is necessary for EVP synthesis appears to take place at or near the organic-water interface. Once the VP transfer is complete, the slightly acidic 2-pyrrolidone by-product drifts into the basic aqueous phase and EVP moves to the organic medium. In fact, both the strong base and other salts are present overwhelmingly in the aqueous layer during the process. The conversion of 2-pyrrolidone to 4-AB in the presence of aqueous base reduces the concentration of base in the organic phase, thereby avoiding an undesired further reaction of EVP to ethylidene-bis-vinylpyrrolidone (EVBP).

The yield of isomeric EVP obtained in the process of the invention is about 5-30% based on reacted VP.

The invention will now be illustrated with reference to the following examples.

EXAMPLE 1

A 1-1 reaction vessel equipped with a reflux condenser and a mechanical stirrer was charged with 100 g. of vinylpyrrolidone (VP) monomer and 300 g. of B.F. Goodrich Caustic 20 solution (20% NaOH). The 2-phase reaction mixture was given a blanket of nitrogen and heated to 100°C. where it was held for 5 hours while stirring vigorously at 800 rpm. Then the reaction product was extracted with chloroform and the organic layer (the bottom layer) was collected and washed several times with a saturated Nacl solution until the final aqueous washing was neutral. The chloroform was removed by rotary evaporation at room temperature. Thereafter, the organic product was fractionally distilled at 60-90° at 1.33 x $10^2$ Pa (1 mm Hg) to remove 20 g of unreacted vinylpyrrolidone monomer, and the 2-pyrrolidone by-product. Then at 80-90°C. a solid product was collected which was recrystallized from ice water and dried in a desiccator. A total of 9.3 g of white, needle-shaped crystals were obtained having a sharp melting point of 59-61°C. The isomeric compound was identified as 1-vinyl-3(E)-ethylidene pyrrolidone having a purity of > 95% by GC/MS$^1$H and $^{13}$C NMR analysis. The yield was 11.6% based upon the amount of VP reacted.

$$\% \text{ EVP Yield} = \frac{9.3 \text{ [EVP]}}{[100 \text{ (Initial VP)} - 20 \text{ (Unreacted VP)}]} \text{ X } 100$$

EXAMPLE 2

The procedure of Example 1 was followed using a reaction mixture of 150 g. of VP, 180 g of a 50 wt. % NaOH solution and 270 g. of distilled water. A total of 8.7 g. of the isomeric compound was obtained. The yield was 7.3%.

EXAMPLES 3-5

The procedure of Example 2 was followed using reaction periods of 7, 14 and 21 hours. The respective yields were 9.7%, 18.5%, 28%.

EXAMPLES 6-7

A stainless steel Buchi reactor was used as the reaction vessel at an initial pressure of 3 x $10^3$ kPa (3 bars) of nitrogen pressure and at room temperature. The reaction was carried out at 140°C. for 2 hours using 320 g. of VP, 40 g of 50% NaOH solution and 40 g. of distilled water (Ex. 6), and 320 g. of VP and 80 g. of 50% NaOH solution (Ex. 7), to provide yields of 25%, and 28%, respectively, of the isomeric compound.

EXAMPLE 8

A reaction mixture of 80% VP and 5% NaOH was heated at 140°C. for 2 hours under 3 x $10^2$ kPa (3 bars) of $N_2$ pressure (14 ppm $O_2$ only). The organic reaction product (top layer) was separated from the aqueous phase; it was found to contain 75% VP and about 19% of the isomeric (E) compound based on the total mixture of the top layer.

The top layer was then flash distilled at 75-95°C. under 1.33 x $10^2$ Pa (1 mm Hg) and VP and 2-pyrrolidone was collected in the receiving flask. The isomeric compound vapor was distilled at 80-90°C. and solidified on the condenser. The condenser then was heated to 70°C. and isomeric compound was collected as a liquid in the receiving flask. Upon cooling to a solid, the isomeric compound was recrystallized in the cold from a mixture of acetone/water. The yield of isomeric EVP was 26%.

The isomeric EVP compound of the invention finds utility as a crosslinking agent in the direct polymerization of vinylpyrrolidone to crosslinked polyvinylpyrrolidone (XL PVP) at low temperatures. For this purpose a polymerizable reaction solution containing about 50-96% by weight VP and 4-30% by weight (E) EVP is formed in situ, as described above, and polymerized to XL PVP.

Crosslinked polyvinylpyrrolidone may be produced from the polymerizable composition by the steps which comprise: diluting the polymerizable composition with added vinylpyrrolidone and water to form a predetermined reaction solution, and heating the solution at about 80-120°C. in an inert atmosphere, under agitation, in the absence of base. The crosslinked polyvinylpyrrolidone polymer product is obtained within about 1-3 hours of such heating, and can be recovered easily without requiring extensive washing to remove caustic and other salts as in the conventional PVP process.

EXAMPLE 9

62.7 g. of a colorless, purified polymerizable composition containing 92% by weight VP and 8% by weight EVP (E isomer) was diluted with 57.3 g. vinylpyrrolidone and 20 g. of water to form a reaction solution of 80% vinylpyrrolidone, 2.5% EVP and 17.5% water. The reaction solution was charged into a Buchi reactor at 0.3 x $10^2$ kPa (0.3 bar) nitrogen pressure (< 18 ppm $O_2$), under 800 rpm agitation, and heated for 2 hours at 100°C. The product was crosslinked polyvinylpyrrolidone in an amount of 108 g. (90 % yield).

XL PVP also may be produced by a one-step process using isomeric EVP as an added crosslinker instead of being made in situ. Accordingly, the invention provides a one-step process for the polymerization of vinylpyrrolidone in aqueous solution in the presence of a predetermined amount of an added isomeric EVP as crosslinker, in the absence of base, and at temperatures lower than 140°C., preferably 60-120°C., and, most preferably, about 100°C. The reaction mixture in the process suitably comprises about 1-5% by weight of the added crosslinker, and about 75-85% of vinylpyrrolidone, the rest being water. The crosslinked polyvinylpyrrolidone product made herein had a swell volume of < 50 ml/10 g. of polymer, and was substantially free of side reaction products.

EXAMPLE 10

ONE-STEP POLYMERIZATION

A reaction mixture of 80 g. vinylpyrrolidone, 2.56 g. of powdered isomeric compound crosslinker, as prepared above, and 17.44 g. of water was heated to 100°C. and agitated under an inert atmosphere for 1 hour. The polymerized product was crosslinked polyvinylpyrrolidone which had a swell volume of 40 ml/10 g. of polymer and was substantially colorless. The yield was 90%.

## Claims

1. A process for obtaining the compound 1-vinyl-3(E)-ethylidene pyrrolidone having the formula:

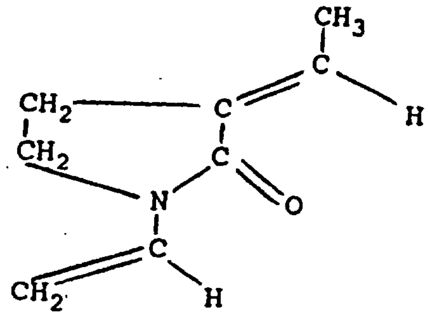

which comprises:

(a) providing a two-phase reaction mixture comprising an organic phase of vinylpyrrolidone in an amount of 25-90% by weight of the reaction mixture, and an aqueous phase which is a solution containing 2-50% by weight of sodium hydroxide, potassium hydroxide or tetraalkyl ammonium hydroxide under vigorous agitation, in an inert atmosphere,
(b) heating said two-phase mixture at 120-170°C for 0.5-10 hours to produce a reaction product with an organic layer and
(c) recovering said compound from the reaction product by fractional distillation of the organic layer under vacuum.

2. A process according to claim 1 wherein, in (c) the organic layer is separated from the aqueous layer, and the organic layer is fractionally distilled under vacuum first at 75-90° at 2.66 x $10^2$Pa (2 mm Hg) to remove unreacted vinylpyrrolidone, the compound being obtained as a colourless solid upon further distillation at 80-90°C at 1.33 x $10^2$Pa (1 mm Hg).

3. A process for obtaining the compound 1-vinyl-3(E)-ethylidene pyrrolidone having the formula

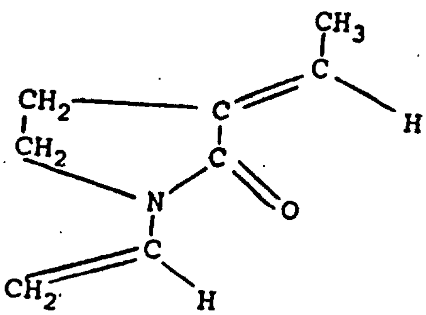

which comprises:

(a) providing a two-phase reaction mixture comprising an organic phase of vinylpyrrolidone in an amount of 25-90% by weight of the reaction mixture, and an aqueous phase which is a solution containing 2-50% by weight of sodium hydroxide, potassium hydroxide or tetraalkyl ammonium hydroxide under vigorous agitation, in an inert atmosphere,
(b) heating said two-phase mixture at 120-170°C for 0.5-10 hours to produce a reaction product with an organic layer and
(c) recovering said compound from the reaction product by extracting the reaction product with a volatile organic solvent, and fractionally distilling the extract under vacuum.

4. A process according to claim 3 wherein the organic solvent extract is distilled successively to remove the solvent, then at 60°-90° at 1.33-2.66 x $10^2$Pa (1-2 mm Hg) to remove unreacted vinylpyrrolidone and then at 80°-90°C to obtain the compound as a colourless solid.

5. A process according to claim 2 or claim 4 wherein the solid is recrystallized from water or water/acetone to yield white, needle-shaped crystals having a melting point of 59-61°C.

**Patentansprüche**

1. Verfahren zum Erhalt der Verbindung 1-Vinyl-3(E)-ethylidenpyrrolidon der Formel:

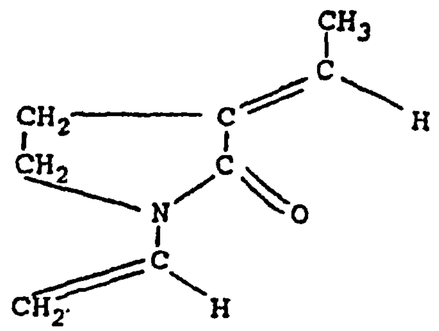

welches umfasst:

(a) das Bereitstellen eines zweiphasigen Reaktionsgemischs, das eine organische Phase aus Vinylpyrrolidon in einer Menge von 25 bis 90 Gew.-% des Reaktionsgemischs und eine wässrige Phase umfasst, die eine Lösung ist, die 2 bis 50 Gew.-% Natriumhydroxid, Kaliumhydroxid oder Tetraalkylammoniumhydroxid enthält, unter heftigem Rühren in einer Inertatmosphäre,
(b) das Erhitzen des zweiphasigen Reaktionsgemischs auf 120 bis 170 °C für 0,5 bis 10 h, um ein Reaktionsprodukt mit einer organischen Phase zu erzeugen, und
(c) das Gewinnen der Verbindung aus dem Reaktionsprodukt durch fraktionierte Destillation der organischen Phase im Vakuum.

2. Verfahren nach Anspruch 1, worin unter (c) die organische Phase von der wässrigen Phase abgetrennt wird und die organische Phase im Vakuum zunächst bei 75 bis 90° und 2,66 x $10^2$ Pa (2 mmHg) fraktioniert destilliert wird, um nichtumgesetztes Vinylpyrrolidon zu entfernen, wobei die Verbindung bei weiterer Destillation bei 80 bis 90 °C und 1,33 x $10^2$ Pa (1 mmHg) als farbloser Feststoff erhalten wird.

3. Verfahren zum Erhalt der Verbindung 1-Vinyl-3(E)-ethylidenpyrrolidon der Formel:

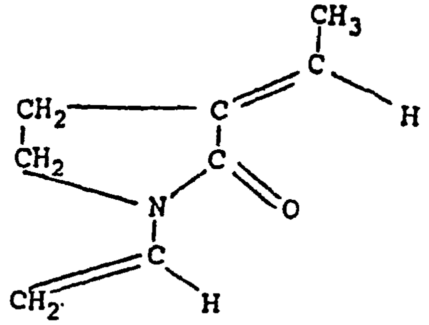

welches umfasst:

(a) das Bereitstellen eines zweiphasigen Reaktionsgemischs, das eine organische Phase aus Vinylpyrrolidon in einer Menge von 25 bis 90 Gew.-% des Reaktionsgemischs und eine wässrige Phase umfasst, die eine Lösung ist, die 2 bis 50 Gew.-% Natriumhydroxid, Kaliumhydroxid oder Tetraalkylammoniumhydroxid enthält, unter heftigem Rühren in einer Inertatmosphäre,
(b) das Erhitzen des zweiphasigen Reaktionsgemischs auf 120 bis 170 °C für 0,5 bis 10 h, um ein Reaktionsprodukt mit einer organischen Phase zu erzeugen, und
(c) das Gewinnen der Verbindung aus dem Reaktionsprodukt durch Extrahieren des Reaktionsprodukts mit einem flüchtigen organischen Lösungsmittel und fraktionierte Destillation des Extrakts im Vakuum.

**4.** Verfahren nach Anspruch 3, worin der organische Lösungsmittel-Extrakt nacheinander zur Entfernung des Lösungsmittels, dann bei 60 bis 90 °C und 1,33 bis 2,66 x $10^2$ Pa (1-2 mmHg), um nichtumgesetztes Vinylpyrrolidon zu entfernen, und dann bei 80 bis 90 °C destilliert wird, was die Verbindung als farblosen Feststoff ergibt.

**5.** Verfahren nach Anspruch 2 oder 4, worin der Feststoff aus Wasser oder Wasser/Aceton umkristallisiert wird, was weiße, nadelförmige Kristalle mit einem Schmelzpunkt von 59 bis 61 °C ergibt.

## Revendications

**1.** Procédé pour l'obtention du composé 1-vinyl-3 (E)-éthylidène pyrrolidone ayant la formule :

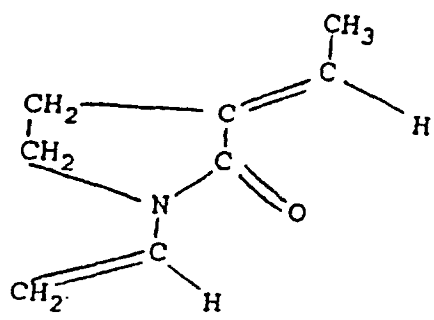

qui consiste à :

(a) produire un mélange réactionnel biphasé comprenant une phase organique de vinylpyrrolidone en une quantité de 25-90% en poids du mélange réactionnel et une phase aqueuse qui est une solution contenant 2-50% en poids d'hydroxyde de sodium, d'hydroxyde de potassium ou d'hydroxyde des tétraalkyl ammonium sous agitation vigoureuse, dans une atmosphère inerte,
(b) chauffer ledit mélange biphasé à 120-170°C pendant 0,5-10 heures pour produire un produit réactionnel avec une couche organique et
(c) récupérer ledit composé du produit réactionnel par distillation fractionnée de la couche organique sous vide.

**2.** Procédé selon la revendication 1 où, en (c) la couche organique est séparée de la couche aqueuse et la couche organique subit une distillation fractionnée sous vide, d'abord à 75-90° à 2,66 x $10^2$Pa (2 mm Hg) pour éliminer la vinylpyrrolidone n'ayant pas réagi, le composé étant obtenu sous la forme d'un solide incolore lors d'une plus ample distillation à 80-90°C à 1,33 x $10^2$Pa (1 mm Hg).

**3.** Procédé pour l'obtention du composé 1-vinyl-3(E)-éthylidène pyrrolidone ayant la formule

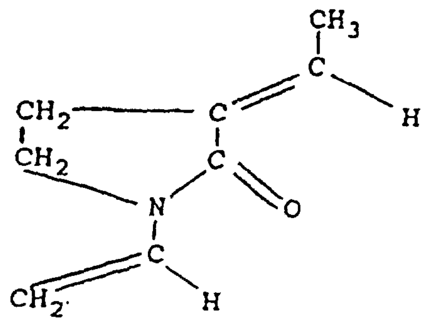

qui consiste à :

(a) produire un mélange réactionnel biphasé comprenant une phase organique de vinylepyrrolidone en une quantité de 25-90% en poids du mélange réactionnel et une phase aqueuse qui est une solution contenant 2-50% en poids d'hydroxyde de sodium, d'hydroxyde de potassium ou d'hydroxyde de tétraalkyl ammonium sous agitation vigoureuse dans une atmosphère inerte,
(b) chauffer ledit mélange biphasé à 120-170°C pendant 0,5-10 heures pour produire une produit réactionnel avec une couche organique et
(c) récupérer ledit composé du produit réactionnel par extraction du produit réactionnel avec un solvant organique volatil et faire subir un distillation fractionnée à l'extrait sous vide.

**4.** Procédé selon la revendication 3 où l'extrait dans le solvant organique est distillé en succession pour éliminer le solvant, puis à 60°-90° à 1,33-2,66 x $10^2$Pa (1-2 mm Hg) pour éliminer la vinylpyrrolidone n'ayant pas réagi puis à 80°-90°C pour obtenir le composé sous la forme d'un solide incolore.

**5.** Procédé selon la revendication 2 ou la revendication 4 où le solide est recristallisé dans l'eau ou de l'eau/acétone pour donner des cristaux blancs aciculaires ayant un point de fusion de 59-61°C.